# EUROPÄISCHE PATENTSCHRIFT

(11) **EP 2 167 909 B1**
(45) Veröffentlichungstag und Bekanntmachung des Hinweises auf die Patenterteilung: **25.05.2011**
(21) Anmeldenummer: 08786156.3
(22) Anmeldetag: 15.07.2008
(51) Int. Cl.: G01B 11/16, A61B 5/11

(54) **OPTISCHER FASERSENSOR MIT ELEKTRISCHEN ANSCHLÜSSEN**
OPTICAL FIBER SENSOR HAVING ELECTRICAL CONNECTORS
CAPTEUR À FIBRES OPTIQUES COMPORTANT DES CONNEXIONS ÉLECTRIQUES

(30) Priorität: 18.07.2007 DE 102007034264; 21.09.2007 DE 102007046385
(43) Veröffentlichungstag der Anmeldung: 31.03.2010
(73) Patentinhaber: Siemens Aktiengesellschaft, 80333 München (DE)
(72) Erfinder: BARBOUTIS, Grigorios, 10589 Berlin (DE); GOLDBECK, Dirk David, 81927 München (DE); HAPPEL, Tobias, 10553 Berlin (DE); KWIATEK, Andre Matthias, 12159 Berlin (DE); NERRETER, Stefan, 15754 Heidesee OT Blossin (DE)
(74) Vertreter: Wolff, Harry
(86) Internationale Anmeldenummer: PCT/EP2008/059254
(87) Internationale Veröffentlichungsnummer: WO 2009/010519

(56) Entgegenhaltungen:
- WO-A-99/07659
- JP-A- 7 203 605
- US-A1- 2002 024 656

## Beschreibung

Die Erfindung betrifft einen optischen Fasersensor mit mindestens einer optischen Sensorfaser, welche an ihren Enden mit einer optischen Sendereinheit zur Einspeisung eines Messsignals und mit einer optischen Empfängereinheit zur Registrierung dieses Messsignals ausgestattet ist, wobei die Sendereinheit und die Empfängereinheit außerdem elektrische Anschlüsse aufweisen. Sendereinheit und Empfängereinheit sind räumlich voneinander getrennt, d. h. jeweils eigenständige Baueinheiten.

Ein Fasersensor der eingangs angegebenen Art ist beispielsweise in der US 6,940,062 B2 beschrieben. Dieser optische Fasersensor kann beispielsweise zur Ermittlung von Verformungen verwendet werden, wobei die Applikation des optischen Fasersensors derart erfolgt, dass die Verformung eines Bauteils, auf dem der optische Fasersensor angebracht ist, eine Verbiegung der optischen Sensorfaser des optischen Fasersensors bewirkt. Diese lässt sich aufgrund des Einflusses der Verbiegung auf das optische Dämpfungsverhalten der Sensorfaser nachweisen. Zu diesem Zweck wird ein Messsignal von einer optischen Sendereinheit in die optische Sensorfaser eingespeist und das Messsignal am anderen Ende der optischen Sensorfaser durch eine Empfängereinheit ausgewertet. Die Lichtintensität des empfangenden Messsignals lässt einen Rückschluss auf den Biegungszustand der Sensorfaser zu. Über Steckkontakte können die optische Sendereinheit und die optische Empfängereinheit jeweils mit Energie versorgt werden bzw. es kann die Messgröße elektrisch ausgelesen werden.

Der Aufbau des Fasersensors gemäß der US 6,940,062 B2 kann bei bestimmten Anwendungsfällen jedoch Probleme aufwerfen. Beispielsweise ist gemäß der EP 968 400 B1 ein Anwendungsfall für einen optischen Fasersensor beschrieben, bei dem die Bewegungen des menschlichen Körpers überwacht werden sollen. Zu diesem Zweck wird der optische Fasersensor am menschlichen Körper befestigt. Hierbei schränken jedoch elektrische Anschlüsse und die dort angebrachten Verbindungsleitungen an beiden Enden der Sensorfaser die Bewegungsfreiheit des Probanten ein, weswegen die ermittelten Messergebnisse in ihrer Aussagekraft begrenzt sind.

Daher wird gemäß der EP 968 400 B1 vorgeschlagen, die Sendereinheit und die Empfängereinheit in einem Gehäuse zu vereinen. Hierdurch entsteht die Möglichkeit, den optischen Fasersensor nur an einem Ende mit der elektrischen Kontaktierung zu versehen. Um die Sendeeinheit und die Empfängereinheit zusammenführen zu können, werden die optischen Fasersensoren in dem Sensorband in Schleifen verlegt, so dass der Anfang und das Ende der jeweiligen Sensorfaser an einem Ende des Sensorbandes liegen. Hierbei wird hingenommen, dass durch diese Maßnahme das Sensorband selbst in seinem Querschnitt doppelt so groß ausfällt, als dies bei einer Führung der Sensorfaser von einem Ende des Sensorbandes zum anderen Ende des Sensorbandes der Fall wäre. Der hiermit verbundene Verlust an Tragekomfort wiegt nämlich die umständliche Kontaktierung an beiden Enden auf.

Gemäß der WO 88/07659 wird ein optischer Fasersensor beschrieben, der zur Ermittlung der Fingerkrümmung an einer menschlichen Hand verwendet werden kann. Dieser Fasersensor ist an den Enden mit einem optischen Sender und einem optischen Empfänger gekoppelt, welche mit einer Verbindungsleitung verbunden sind. Dieser Aufbau des Fasersensors kann entlang der Finger auf einem Handschuh angebracht werden, den der Benutzer dann anziehen kann. Insbesondere kann auch ein zweischaliger Handschuh verwendet werden, wobei der optische Fasersensor zwischen der Innenschale und der Außenschale angebracht ist.

Die Aufgabe der Erfindung liegt darin, einen optischen Fasersensor anzugeben, dessen Tragekomfort und Bedienungskomfort vergleichsweise hoch ist.

Diese Aufgabe wird mit dem eingangs angegebenen Fasersensor erfindungsgemäß dadurch gelöst, dass in dem Fasersensor parallel zur optischen Sensorfaser zumindest eine elektrische Leitung geführt wird, die zumindest einen Teil der elektrischen Anschlüsse der Empfängereinheit mit zumindest einem Teil der Anschlüsse der Sendereinheit verbindet. Als elektrische Leitung im Zusammenhang mit der hier vorgestellten Erfindung soll allgemein eine Anordnung zur Leitung elektrischer Signale oder Versorgungsströme verstanden werden. Dabei kann die elektrische Leitung einadrig oder mehradrig sein, d. h. in einer Leitung können sowohl mehrere elektrische Signale wie auch Versorgungsströme transportiert werden. Durch Vorsehen der elektrischen Leitung parallel verlaufend zur Sensorfaser wird es möglich, einerseits auf eine Rückführung aller optischen Sensorfasern zu einem einzigen Gehäuse zu verzichten und an beiden Enden der Sensorfaser räumlich voneinander unabhängige Einheiten zum Senden und Empfangen der Messsignale (Sendereinheit und Empfängereinheit) zu verwenden. Eine aufwendige Kontaktierung beider Einheiten kann jedoch dadurch vereinfacht werden, dass zwischen der Sendereinheit und der Empfängereinheit eine elektrische Leitung verlegt ist, mit deren Hilfe Kontaktierungen, die für die eine Einheit gedacht sind, an Kontakte der anderen Einheit verlegt werden können. Gemäß einer Alternative des Erfindungsgedankens ist vorgesehen, dass die mindestens eine optische Sensorfaser in ein Sensorband integriert, insbesondere eingebettet, ist. Die Einbettung in ein Sensorband ermöglicht vorteilhaft eine einfache Handhabung des optischen Fasersensors. Zum einen stellt das Sensorband einen gewissen Schutz für die empfindlichen optischen Sensorfasern zur Verfügung. Zum anderen können mehrere Sensorfasern in definierter Lage zueinander in dem Sensorband zusammengefasst werden. Bei dieser Alternative des Sensorbandes ist außerdem vorgesehen, dass die elektrische Leitung als Strangleiter ausgebildet ist und ebenfalls in das Sensorband integriert, insbesondere eingebettet, ist. Das Sensorband kann dann vorteilhaft leicht verlegt werden, um eine Messung im gewünschten Anwendungsfall durchzuführen. Dabei muss nicht gesondert auf die optischen bzw. elektrischen Stränge geachtet werden. Hierbei ist es besonders vorteilhaft, wenn die elektrische Leitung im Wesentlichen denselben Durchmesser aufweist wie die mindestens eine optische Faser. Dadurch lässt sich diese fertigungstechnisch einfach gemeinsam mit den optischen Sensorfasern oder der optischen Sensorfaser verlegen und zu einem Sensorband zusammenfassen. Das fertig gestellte Sensorband kann dann insbesondere eine einheitliche Bauhöhe aufweisen, die auch im Bereich der elektrischen Leitung der Bauhöhe des Bereiches mit den vorzugsweise mehreren Sensorfasern entspricht.

Eine andere Alternative besteht erfindungsgemäß darin, dass die elektrische Leitung als Bandleiter ausgeführt ist. Ein Bandleiter hat vorteilhaft eine sehr geringe Bauhöhe, so dass dieser auf einfache Weise parallel zu der Sensorfaser geführt werden kann, ohne dass der durch das Sensorband eingenommene Bauraum wesentlich vergrößert wird.

Dabei ist es gemäß einer Unteralternative besonders vorteilhaft, wenn der Bandleiter und das Sensorband Seite an Seite angeordnet sind. Hiermit ist gemeint, dass die Bänder jeweils mit der breiten Seite des Bandes einander benachbart liegen, also nicht Kante an Kante sondern übereinander verlegt werden. Hierdurch lässt sich vorteilhaft durch die große zur Verfügung stehende Fügefläche ein fester Verband erzeugen. Gleichzeitig kann der Bandleiter dabei die Sensorfasern mechanisch stützen. Der Bandleiter und das Sensorband können beispielsweise mit einer Klebeschicht miteinander verbunden sein. Dies ist fertigungstechnisch insbesondere bei Kleinserien besonders einfach durchzuführen. Die Klebeschicht kann auf eines der Bänder aufgebracht werden. Es ist jedoch auch möglich, ein doppelseitiges Klebeband zu verwenden.

Eine andere Unteralternative besteht darin, dass die elektrische Leitung durch direkte Herstellung von Leitpfaden auf dem Sensorband als Bandleiter ausgeführt ist. Hierbei können die in der Fertigung von elektrischen Leitpfaden üblichen Verfahren zur Anwendung kommen. Denkbar sind beispielsweise photomechanische Verfahren, bei denen nach einer geeigneten Strukturierung der Bandoberfläche die Leitpfade mittels Ätzen hergestellt werden. Eine andere Möglichkeit besteht darin, unter Einsatz von Schablonen die Leitpfade auf dem Sensorband durch Beschichten herzustellen. In jedem Fall wird durch die direkte Herstellung der Leitpfade auf dem Sensorband eine besonders raumsparende Lösung geschaffen.

Erfindungsgemäß ist bei beiden Unteralternativen vorgesehen, dass der Verbund aus Sensorband und Bandleiter mit einer Ummantelung umhüllt ist. Diese Ummantelung stellt einen zusätzlichen Schutz des gesamten Verbandes dar, insbesondere bei der direkten Herstellung der Leitpfade auf dem Sensorband führt die Ummantelung zusätzlich zu einer elektrischen Isolierung, die vorteilhaft die Einsatzmöglichkeiten des erzeugten Fasersensors erweitert.

Eine vorteilhafte Ausgestaltung der Erfindung wird erhalten, wenn die Sendereinheit oder die Empfängereinheit ausschließlich elektrische Anschlüsse aufweist, die über die elektrische Leitung verbunden sind. Damit ist zumindest eine der Einheiten vorteilhaft völlig frei von externen elektrischen Anschlüssen, so dass diese Einheit dann nicht mit externen elektrischen Verbindungsleitungen kontaktiert werden muss. Vielmehr verlaufen alle elektrischen Kontaktleitungen, die zum Betrieb der betreffenden Einheit notwendig sind, über die elektrische Leitung, die parallel zur optischen Sensorfaser verläuft. Hierdurch wird der Tragekomfort wesentlich verbessert, weil eine elektrische Kontaktierung nur an einer der Einheiten (Sendereinheit oder Empfängereinheit) notwendig ist. Weiterhin wird auch der Tragekomfort der Sensorfaser, die z. B. in ein Sensorband integriert sein kann, durch die zusätzliche Anwesenheit einer weiteren elektrischen Leitung nur unwesentlich beeinträchtigt. Diese elektrische Leitung kann Signalleitungen für mehrere optische Sensorfasern aufweisen, da der hierzu notwendige Querschnitt geringer als bei den optischen Sensorleitungen ausfällt.

Eine vorteilhafte Ausgestaltung der Erfindung wird erhalten, wenn die Sendereinheit und die Empfängereinheit ausschließlich elektrische Anschlüsse aufweisen, die über die elektrische Leitung verbunden sind. Diese Ausgestaltung der Erfindung setzt voraus, dass der optische Fasersensor autark funktioniert. Dies bedeutet, dass der Fasersensor einerseits eine Energiequelle zum Betrieb aufweisen muss und andererseits eine drahtlose Schnittstelle zum Auslesen der Messdaten zur Verfügung stellen muss oder einen Speicher für diese Daten aufweisen muss, damit diese nach abgeschlossener Messung ausgewertet werden können. In diesem Fall kann zum Auslesen eine elektrische Kontaktierung vorgesehen sein, die vorteilhaft während der Messung nicht angeschlossen sein muss. Bei einem autark funktionierenden optischen Fasersensor hat die Verlegung einer elektrischen Leitung parallel zur optischen Sensorfaser den Vorteil, dass die Bauteile, die zum autarken Funktionieren des Fasersensors notwendig sind, jeweils nur einmal vorgesehen werden müssen. Beispielsweise kann die Sendereinheit die elektrische Spannungsquelle beherbergen, wobei über die elektrische Leitung auch eine elektrische Versorgung der Empfängereinheit möglich ist. Soll der Speicherbaustein für die Messwerte bzw. eine drahtlose Schnittstelle zur Übermittlung derselben auch in der Sendereinheit vorgesehen sein (z. B. um die Empfängereinheit möglichst klein zu gestalten), so müssen auch Signalleitungen zwischen der Empfängereinheit und der Sendereinheit verlegt sein.

Weitere Einzelheiten der Erfindung werden nachfolgend anhand der Zeichnung erläutert. Gleiche oder sich entsprechende Zeichnungselemente in den einzelnen Figuren sind jeweils mit den gleichen Bezugszeichen versehen und werden nur insoweit mehrfach erläutert, wie sich Unterschiede zwischen den einzelnen Figuren ergeben. Es zeigen
- Figur 1: einen optischen Fasersensor im Längsschnitt nach dem Stand der Technik schematisch,
- Figur 2: die Aufsicht auf ein Ausführungsbeispiel des erfin- dungsgemäßen Fasersensors, welcher auf einem Trä- gerband befestigt ist und
- Figur 3 bis 5: Querschnitte durch die Sensorbänder unterschiedlicher Ausführungsbeispiele des er- findungsgemäßen Fasersensors.

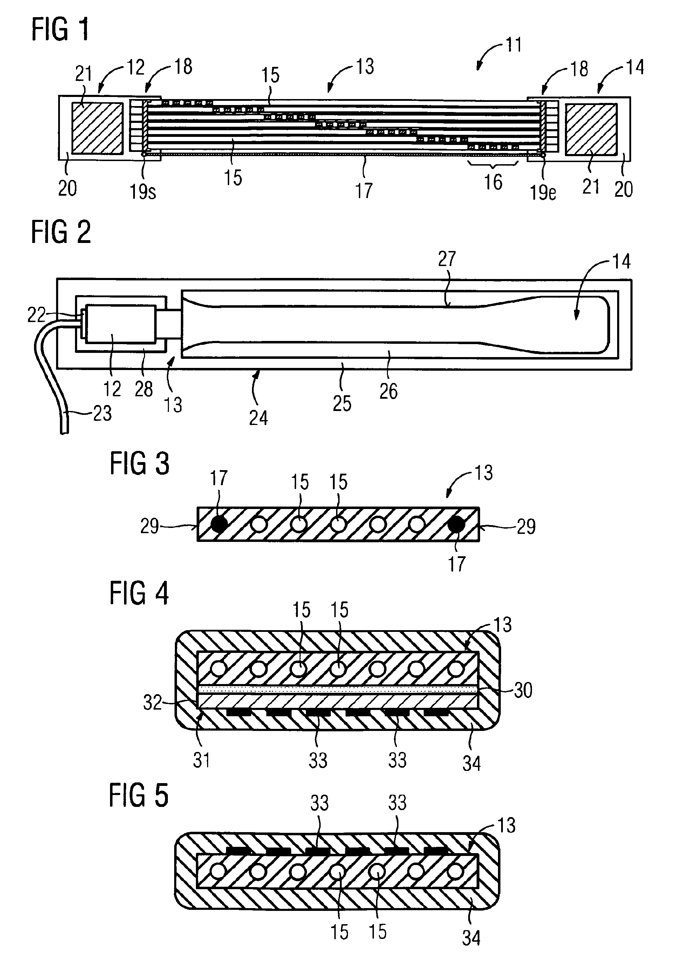

Ein optischer Fasersensor 11 gemäß Figur 1 besteht aus drei Baueinheiten, einer optischen Sendereinheit 12, einem Sensorband 13 und einer optischen Empfängereinheit 14. Die Sendereinheit 12 ist am einen Ende des Sensorbandes angebracht und die räumlich von der Sendereinheit getrennte Empfängereinheit 14 ist am anderen Ende des Sensorbandes 13 angebracht.

Das Sensorband weist mehrere optische Sensorfasern 15 auf, die jeweils an unterschiedlichen Stellen des Sensorbandes biegesensitive Abschnitte 16 aufweisen. Auf diese Weise ist eine ortsaufgelöste Ermittlung der Biegung des Sensorbandes 13 möglich. Parallel zu den Sensorfasern verläuft weiterhin eine als Strangleiter 17 ausgebildete elektrische Leitung. Die Sensorfasern 15 sind in der Empfängereinheit 14 und der Sendereinheit 12 über optische Schnittstellen 18 optisch kontaktiert. Weiterhin weisen die Sendereinheit 12 und die Empfängereinheit 14 elektrische Anschlüsse 19e und 19s auf, über die eine Kontaktierung des Strangleiters möglich ist. Diese sind in Figur 1 nur schematisch dargestellt. Sollte der Strangleiter mehradrig ausgeführt sein, sind selbstverständlich auch mehrere Anschlüsse 19e, 19s notwendig, die in Figur 1 der besseren Übersichtlichkeit halber weggelassen wurden.

Bei der Lösung für den optischen Fasersensor gemäß Figur 1 stellt dieser ein autarkes System dar. Die Sendereinheit 12 und Empfängereinheit 13 bestehen jeweils aus Leiterplatten 20, auf denen eine Schutzkappe 21 vorgesehen ist. Die Schutzkappe dient als Gehäuse für jeweilige Treiberelektroniken, eine Spannungsversorgung und ein Funkmodul zur kabellosen Weitergabe der Messwerte sowie zum Empfang von Steuersignalen für den optischen Fasersensor. Diese Bauelemente sind jedoch nicht näher dargestellt.

Bei dem Fasersensor gemäß Figur 2 ergeben sich im Vergleich zu Figur 1 folgende Unterschiede. Anders als bei dem Sensorband 13 gemäß Figur 1 ist bei dem Sensorband 13 gemäß Figur 2 auch der elektrische Leiter (als Strangleiter ausgebildet und nicht dargestellt) in das Sensorband 13 eingebettet. Gemäß Figur 1 verläuft der Strangleiter 17 parallel neben dem Sensorband 13 entlang. Auch die Sendereinheit 12 und die Empfängereinheit 14 sind jeweils vollständig in ein Gehäuse integriert. Außerdem besitzt die Sendereinheit 12 zusätzlich nicht näher dargestellte elektrische Anschlüsse, die mit einem Stecker 22 kontaktiert werden können. Hierdurch lässt sich eine Versorgungs- und Signalleitung 23 an die Sendereinheit 12 anschließen. Die elektrische Versorgung der Empfängereinheit 14 sowie die Übertragung von Signalen zwischen Sendereinheit 12 und Empfängereinheit 14 erfolgt über den nicht dargestellten Strangleiter (vgl. analog 17 in Figur 1), so dass eine externe Kontaktierung der Empfängereinheit 14 nicht notwendig ist.

Da die Empfängereinheit 14 frei von externen Kontakten ist, kann der optische Fasersensor gemäß Figur 2 auf einem Trägerband 24 montiert werden. Dieses besteht aus einer flexiblen Unterlage 25, die beispielsweise bei der Applikation des Fasersensors als Rückensensor auf der Haut eines Probanden festgeklebt werden kann. Hierbei kommt ein hautverträglicher Kleber zum Einsatz. Aufgrund der Flexibilität der Unterlage ist ein hoher Tragekomfort gewährleistet, da das Trägerband die Bewegungen der Wirbelsäule und die damit verbundenen elastischen Veränderungen der Haut nachvollziehen kann. Auf der Unterlage 25 ist weiterhin eine elastische Decklage 26 derart aufgebracht, dass sich eine einseitig offene Tasche ergibt. In diese kann der Fasersensor eingeschoben werden, wobei sich dessen Kontur 27 unter der elastischen Decklage abzeichnet. Die Empfängereinheit 14 befindet sich dabei am Ende der Tasche. Die Sendereinheit 12 wird auf einer starren Fixierungsplatte 28 befestigt, so dass sich ein Referenzpunkt auf dem Trägerband 24 für den Fasersensor ergibt.

Der Figur 3 lässt sich ein Querschnitt durch das Sensorband 13 entnehmen, wie es beispielsweise bei dem Fasersensor gemäß Figur 2 zum Einsatz kommen könnte. Die Strangleiter 17, von denen zwei vorgesehen sind, sind an den beiden Kanten 29 angeordnet und schließen somit die Sensorfaser 15 zwischen sich ein. Dies hat den Vorteil, dass die im Vergleich zu den Strangleitern 17 empfindlicheren Sensorfasern 15 geschützt sind. Die Strangleiter 17 sowie die Sensorfasern 15 sind gemeinsam in das Material des Sensorbandes 13 eingebettet, dies kann beispielsweise durch Eingießen in einen Silikongummi erfolgen, welcher eine hohe Biegsamkeit des gewonnen Sensorbandes 13 gewährleistet.

Gemäß Figur 4 ist eine andere mögliche Bauform des Sensorbandes 13 dargestellt. Dieses weist ausschließlich Sensorfasern 15 auf, die in der zu Figur 3 beschriebenen Weise eingegossen werden können. An der Unterseite des Sensorbandes 13 ist weiterhin eine Klebeschicht 30 angebracht, die das Sensorband 13 mit einem elektrischen Bandleiter 31 verbindet. Der Bandleiter 31 weist ein Substrat 32 auf, auf das Leitpfade 33 beispielsweise durch ätztechnisches Strukturieren hergestellt wurden. Der gesamte Verbund aus Sensorband 13 und Bandleiter 31 ist zusätzlich mit einer elastischen Ummantelung 34 beispielsweise aus Gummi versehen.

Gemäß Figur 5 sind die Leitpfade 33 direkt auf dem Sensorband 13 hergestellt. Dies kann beispielsweise durch Beschichten im CVD-Verfahren erfolgen. Somit ist in das Sensorband 13 gleichzeitig die Funktionalität des Bandleiters 33 gemäß Figur 4 integriert. Auch dieser Verbund ist entsprechend der Ausgestaltung gemäß Figur 4 mit einer Ummantelung 34 versehen.

## Patentansprüche

1. Optischer Fasersensor mit mindestens einer optischen Sensorfaser (15) welche an ihrem einen Ende mit einer optischen Sendereinheit (12) zur Einspeisung eines Messsignals und räumlich hiervon getrennt an ihrem anderen Ende mit einer optischen Empfängereinheit (14) zur Registrierung dieses Messsignals ausgestattet ist, wobei die Sendereinheit (12) und die Empfängereinheit (14) außerdem elektrische Anschlüsse (19e, 19s) aufweisen, wobei in dem Fasersensor parallel zur optischen Sensorfaser (15) zumindest eine elektrische Leitung (17, 31) geführt wird, die zumindest einen Teil der elektrischen Anschlüsse (19e) der Empfängereinheit (14) mit zumindest einem Teil der Anschlüsse (19s) der Sendereinheit (12) verbindet,
**dadurch gekennzeichnet,**
- **dass** die mindestens eine optische Sensorfaser (15) in ein Sensorband (13) eingebettet ist und
- **dass** die elektrische Leitung als Strangleiter (17) ausgebildet ist und ebenfalls in das Sensorband (13) eingebettet ist.

2. Fasersensor nach Anspruch 1,
**dadurch gekennzeichnet,**
**dass** die elektrische Leitung (17) im Wesentlichen denselben Durchmesser (d) aufweist, wie die mindestens eine optische Faser.

3. Optischer Fasersensor mit mindestens einer optischen Sensorfaser (15) welche an ihrem einen Ende mit einer optischen Sendereinheit (12) zur Einspeisung eines Messsignals und räumlich hiervon getrennt an ihrem anderen Ende mit einer optischen Empfängereinheit (14) zur Registrierung dieses Messsignals ausgestattet ist, wobei die Sendereinheit (12) und die Empfängereinheit (14) außerdem elektrische Anschlüsse (19e, 19s) aufweisen, wobei in dem Fasersensor parallel zur optischen Sensorfaser (15) zumindest eine elektrische Leitung (17, 31) geführt wird, die zumindest einen Teil der elektrischen Anschlüsse (19e) der Empfängereinheit (14) mit zumindest einem Teil der Anschlüsse (19s) der Sendereinheit (12) verbindet,
**dadurch gekennzeichnet,**
- dass die mindestens eine optische Sensorfaser (15) in ein Sensorband (13) eingebettet ist,
- dass die elektrische Leitung als Bandleiter (31) ausgeführt ist, wobei der Bandleiter (31) und das Sensorband (13) entweder Seite an Seite angeordnet sind oder der Bandleiter (31) durch direkte Herstellung von Leitpfaden (33) auf dem Sensorband (13) ausgeführt ist, und
- dass der Verbund aus Sensorband (13) und Bandleiter mit einer Ummantelung (34) umhüllt ist.

4. Fasersensor nach einem der vorangehenden Ansprüche,
**dadurch gekennzeichnet,**
**dass** die Sendereinheit (12) oder die Empfängereinheit (14) ausschließlich elektrische Anschlüsse (19e, 19s) aufweist, die über die elektrischen Leitung verbunden sind.

5. Fasersensor nach Anspruch 4,
**dadurch gekennzeichnet,**
**dass** die Sendereinheit (12) und die Empfängereinheit (14) ausschließlich elektrische Anschlüsse (19e, 19s) aufweisen, die über die elektrischen Leitung verbunden sind, wobei der Fasersensor über eine Energiequelle zum Betrieb und eine drahtlose Schnittstelle zum Auslesen der Messdaten verfügt.

## Claims

1. Optical fiber sensor having at least one optical sensor fiber (15) which is equipped at one of its ends with an optical transmitter unit (12) for feeding in a measurement signal, and, physically separated from this at its other end, with an optical receiver unit (14) for registering this measurement signal, wherein the transmitter unit (12) and the receiver unit (14) also have electrical connections (19e, 19s), wherein at least one electrical line (17, 31) is routed in the fiber sensor, parallel to the optical sensor fiber (15) and connects at least some of the electrical connections (19e) of the receiver unit (14) to at least some of the connections (19s) of the transmitter unit (12),
**characterized in that**
- the at least one optical sensor fiber (15) is embedded, in a sensor ribbon (13), and
- **in that** the electrical line is in the form of a line conductor (17), and is likewise embedded, in the sensor ribbon (13).

2. Fiber sensor according to Claim 1,
**characterized**
**in that** the electrical line (17) essentially has the same diameter (d) as the at least one optical fiber.

3. Optical fiber sensor having at least one optical sensor fiber (15) which is equipped at one of its ends with an optical transmitter unit (12) for feeding in a measurement signal, and, physically separated from this at its other end, with an optical receiver unit (14) for registering this measurement signal, wherein the transmitter unit (12) and the receiver unit (14) also have electrical connections (19e, 19s), wherein at least one electrical line (17, 31) is routed in the fiber sensor, parallel to the optical sensor fiber (15) and connects at least some of the electrical connections (19e) of the receiver unit (14) to at least some of the connections (19s) of the transmitter unit (12),
**characterized**
**in that**
- the at least one optical sensor fiber (15) is embedded, in a sensor ribbon (13),
- **in that** the electrical line is in the form of a ribbon conductor (31), wherein the ribbon conductor (31) and the sensor ribbon (13) are either arranged side-by-side or the ribbon conductor (31) is formed on the sensor ribbon (13) by direct production of the conductive paths (33), and
- **in that** the assembly comprising the sensor ribbon (13) and the ribbon conductor is sheathed with a sheath (34).

4. Fiber sensor according to one of the preceding claims,
**characterized**
**in that** the transmitter unit (12) or the receiver unit (14) has exclusively electrical connections (19e, 19s), which are connected via the electrical line.

5. Fiber sensor according to Claim 4,
**characterized**
**in that** the transmitter unit (12) and the receiver unit (14) have exclusively electrical connections (19e, 19s) which are connected via the electrical line, wherein the fiber sensor has a power source for operation and a wire-free interface for reading the measurement data available.

## Revendications

1. Capteur à fibres optiques, comprenant au moins une fibre ( 15 ) optique de capteur, qui est équipée à l'une de ses extrémités d'une unité ( 12 ) optique formant émetteur pour l'injection d'un signal de mesure, et séparément de celle-ci dans l'espace à son autre extrémité, d'une unité ( 14 ) optique formant récepteur pour l'enregistrement de ce signal de mesure, l'unité ( 12 ) formant émetteur et l'unité ( 14 ) formant récepteur ayant en outre des bornes ( 19e, 19s ) électriques, dans lequel dans le capteur à fibres est guidé, parallèlement à la fibre ( 15 ) optique de capteur, au moins une ligne ( 17, 31 ) électrique, qui relie au moins une partie des bornes ( 19e) électriques de l'unité ( 14 ) formant récepteur à au moins une partie des bornes ( 19s ) de l'unité ( 12 ) formant émetteur,
**caractérisé**
- **en ce que** la au moins une fibre ( 15 ) optique de capteur est incorporée dans un ruban ( 13 ) de capteur et
- **en ce que** la ligne électrique est constituée sous la forme d'un conducteur ( 17 ) en cordon et est incorporée également dans le ruban ( 13 ) de capteur.

2. Capteur à fibres suivant la revendication 1,
**caractérisé**
**en ce que** la ligne ( 17 ) électrique a sensiblement le même diamètre ( d ) que la au moins une fibre optique.

3. Capteur à fibres optiques comprenant au moins une fibre ( 15 ) optique de capteur, qui est équipée à l'une de ses extrémités d'une unité ( 12 ) optique formant émetteur pour l'injection d'un signal de mesure, et séparément de celle-ci dans l'espace à son autre extrémité, d'une unité ( 14 ) optique formant récepteur pour l'enregistrement de ce signal de mesure, l'unité ( 12 ) formant émetteur et l'unité ( 14 ) formant récepteur ayant en outre des bornes ( 19e, 19s ) électriques, dans lequel dans le capteur à fibres est guidé, parallèlement à la fibre ( 15 ) optique de capteur, au moins une ligne ( 17, 31 ) électrique, qui relie au moins une partie des bornes ( 19e ) électriques de l'unité ( 14 ) formant récepteur à au moins une partie des bornes ( 19s ) de l'unité ( 12 ) formant émetteur,
**caractérisé**
- **en ce que** la au moins une fibre ( 15 ) optique de capteur est incorporée dans un ruban ( 13 ) de capteur,
- **en ce que** la ligne électrique est réalisée sous la forme d'un conducteur ( 31 ) à ruban, le conducteur ( 31 ) à ruban et le ruban ( 13 ) de capteur étant disposés côte à côte ou le conducteur ( 31 ) à ruban sortant du ruban ( 13 ) de capteur par production directe de voies ( 33 ) conductrices sur le ruban de capteur, et
- **en ce que** l'ensemble composé du ruban ( 13 ) de capteur et du conducteur à ruban est gainé d'une gaine ( 34 ).

4. Capteur à fibres suivant l'une des revendications précédentes,
**caractérisé**
**en ce que** l'unité ( 12 ) formant émetteur ou l'unité ( 14 ) formant récepteur comporte exclusivement des bornes ( 19e, 19s ) électriques qui sont reliées par la ligne électrique.

5. Capteur à fibres suivant la revendication 4,
**caractérisé**
**en ce que** l'unité ( 12 ) formant émetteur et l'unité ( 14 ) récepteur comporte exclusivement des bornes ( 19e, 19s ) électriques qui sont reliées par la ligne électrique, le capteur à fibres disposant d'une source d'énergie pour le fonctionnement et d'une interface sans fil pour la lecture des données de mesure.
